# EUROPÄISCHE PATENTANMELDUNG

(11) **EP 1 658 877 A1**
(43) Veröffentlichungstag der Anmeldung: **24.05.2006**
(21) Anmeldenummer: 05109268.2
(22) Anmeldetag: 06.10.2005
(51) Int. Cl.: A61N 1/14

(54) **Polster für Patientenliegeflächen**

(30) Priorität: 23.11.2004 DE 102004056461
(71) Anmelder: MAQUET GmbH & Co. KG, 76437 Rastatt (DE)
(72) Erfinder: Burg, Gerd, 77815, Bühl (DE); Harrer, Thomas, 76532, Baden-Baden (DE); Pfeuffer, Reinhard, 76477, Elchesheim-Illingen (DE)
(74) Vertreter: Schaumburg, Thoenes, Thurn, Landskron

(57) **Zusammenfassung**

Bei einem Polster für Patientenliegefläche, insbesondere Lagerflächen von O-perationstischen, bestehend aus einem Schaumstoff-Formteil, dessen Oberfläche eine elektrisch leitfähige Schicht trägt, besteht die elektrisch leitfähige Schicht aus einem graphit- oder rußfreien Leitlack.

## Beschreibung

Die Erfindung betrifft ein Polster für Patientenliegeflächen, insbesondere Lagerflächen von Operationstischen, bestehend aus einem Schaumstoff-Formteil, dessen Oberfläche eine elektrisch leitfähige Schicht trägt.

Polster für Patientenliegeflächen werden in der Regel aus einem elastischen Kunststoffschaumstoff hergestellt. Dabei werden die den Schaumstoff bildenden Materialkomponenten in eine geschlossene Form eingeführt, in der sie unter Bildung des Schaumstoffes miteinander reagieren. Bei geeigneter Wahl der Betriebsparameter bildet sich dabei ein sogenannter Integralschaum, bei dem die Außenoberfläche des Schaumstoffformteiles von einer geschlossenen Haut gebildet ist.

Solche Schaumstoffformteile können sich elektrisch aufladen. In einem Operationssaal, oder in Räumen, in denen mit Narkosegasen hantiert wird, die teilweise explosiv sind, müssen solche statischen Aufladungen und die damit verbundene Gefahr einer Funkenbildung vermieden werden. Hierzu ist es bekannt, die Polster mit einer leitfähigen Schicht zu überziehen, die selbst elastisch sein muss. Die Leitfähigkeit dieser Schicht wird in der Regel durch eingelagerte Graphit- oder Rußteilchen sichergestellt. Dies hat den Nachteil, dass man in der Praxis nur schwarze Polster kennt. Dies ist nicht nur vom ästhetischen Gesichtspunkt her unbefriedigend, sondern erschwert es auch, eine Verunreinigung der Polsteroberfläche zu erkennen.

Der Erfindung liegt die Aufgabe zugrunde, ein Polster der eingangs genannten Art anzugeben, das in beliebigen Farben herstellbar ist.

Diese Aufgabe wird erfindungsgemäß dadurch gelöst, dass die elektrisch leitfähige Schicht aus einem graphit- oder rußfreien Leitlack besteht. Dieser Leitlack kann farblos transparent sein, so dass der entsprechend eingefärbte Kunststoff des Formteiles durch die leitfähige Schicht durchscheint. Der Leitlack kann aber auch Farbpigmente enthalten, so dass die Farbe des Polsters nur durch die Lackschicht bestimmt wird. Die Leitfähigkeit kann z.B. durch eingelagerte quasi transparente Partikel einer anorganischen Verbindung oder auch durch leitfähige Kunststoffe gewährleistet werden.

Der Leitlack kann entweder auf das in der Regel aus Polyurethanschaum bestehende Formteil nach dessen Herstellung aufgebracht werden. Gemäß einer anderen Lösung wird auf die Innenwand der Form, die zur Herstellung des Formteiles benutzt wird, vor dem Einbringen der Materialkomponenten eine graphit- oder rußfreie Leitlackschicht aufgetragen. Diese Schicht bleibt dann an dem in der Form gebildeten Schaumstoff-Formteil haften, wenn dieses aus der Form genommen wird. Der Leitlack kann dabei auf die Innenwand der Form aufgesprüht oder aufgestrichen werden.

Als Leitlack für das erfindungsgemäße Polster eignet sich beispielsweise ein hydroxylgruppenhaltiger Kunstharz-Lack, der unter der Typennummer R4206 von der Firma Karl Wörwag, Lack- und Farbenfabrik GmbH & Co. KG, Strohgäustraße 28, 70435 Stuttgart vertrieben wird.

## Patentansprüche

1. Polster für Patientenliegefläche, insbesondere Lagerflächen von Operationstischen, bestehend aus einem Schaumstoff-Formteil, dessen Oberfläche eine elektrisch leitfähige Schicht trägt, **dadurch gekennzeichnet, dass** die elektrisch leitfähige Schicht aus einem graphit- oder rußfreien Leitlack besteht.

2. Polster nach Anspruch 1, **dadurch gekennzeichnet, dass** die Leitlackschicht farblos transparent ist.

3. Polster nach Anspruch 1, **dadurch gekennzeichnet, dass** die Leitlackschicht einen Farbstoff enthält.

4. Polster nach einem der Ansprüche 1 bis 3, **dadurch gekennzeichnet, dass** das Schaumstoff-Formteil aus Polyurethan besteht.

5. Verfahren zur Herstellung eines Schaumstoff-Formteiles für ein Polster nach einem der Ansprüche 1 bis 4, wobei die den Schaumstoff bildenden Materialkomponenten in eine geschlossene Form eingeführt werden, in der sie unter Bildung des Schaumstoffes miteinander reagieren, **dadurch gekennzeichnet, dass** auf die Innenwand der Form vor dem Einbringen der Materialkomponenten ein graphit- oder rußfreier Leitlack aufgetragen wird.

6. Verfahren nach Anspruch 5, **dadurch gekennzeichnet, dass** der Leitlack auf die Form-Innenwand aufgesprüht wird.

7. Verfahren nach Anspruch 5, **dadurch gekennzeichnet, dass** der Leitlack auf die Form-Innenwand aufgestrichen wird.
